# FASCICULE DE BREVET EUROPEEN

(11) **EP 3 436 133 B1**
(45) Date de publication et mention de la délivrance du brevet: **22.03.2023**
(21) Numéro de dépôt: 17720187.8
(22) Date de dépôt: 30.03.2017
(51) Int. Cl.: A61J 1/14, A61J 1/20, A61M 39/00, A61M 39/04

(54) **PORT, SYSTÈME DE DISTRIBUTION COMPRENANT UN TEL PORT, ET PROCÉDÉ DE FABRICATION**
ÖFFNUNG, ABGABESYSTEM MIT SOLCH EINER ÖFFNUNG UND HERSTELLUNGSVERFAHREN
PORT, DISPENSING SYSTEM COMPRISING SUCH A PORT, AND MANUFACTURING METHOD

(30) Priorité: 31.03.2016 FR 1652766
(43) Date de publication de la demande: 06.02.2019
(73) Titulaire: Sartorius Stedim FMT SAS, 13400 Aubagne (FR)
(72) Inventeur: PALLARES, Alain, 13720 Belcodène (FR); BARHOUMI, Ali, Mhamdia Ben Arous, BP 50 (TN); MENIER, Marie-Christine, 13600 La Ciotat (FR)
(74) Mandataire: Novagraaf International SA
(86) Numéro de dépôt international: PCT/FR2017/050729
(87) Numéro de publication internationale: WO 2017/168097

(56) Documents cités:
- WO-A1-2015/061711
- FR-A1- 2 665 633
- GB-A- 928 842
- US-A- 3 030 955
- US-A- 4 303 067

## Description

L'invention concerne les ports, les systèmes de distribution comprenant de tels ports, et leurs procédés de fabrication.

Plus précisément, l'invention concerne un port adapté pour être fixé à une enceinte et adapté pour être traversé par une aiguille utilisée pour le transfert d'un contenu entre l'intérieur de l'enceinte et l'extérieure de l'enceinte, dans le domaine biopharmaceutique et médical.

Jusque-là, pour transférer un fluide entre l'extérieur et l'intérieur d'une enceinte par l'intermédiaire d'une aiguille, on utilisait un port en polymère (type polycarbonate ou Acrylonitrile Butadiène Styrène ABS), munis d'un septum en silicone par exemple obstruant une des extrémités du port, tel que décrit dans la plaquette commerciale pour les produits de la gamme « *total parenteral nutrition bag* » commercialisée par Sartorius. Dans ce cas, l'aiguille traverse le port, notamment le septum, et le port est collé sur l'enceinte.

Le polymère utilisé pour le port ne pouvant en général pas être fixé directement par soudage au film de l'enceinte (poche en d'acétate de vinyle d'éthylène EVA), il est nécessaire d'utiliser un tube en polyéthylène de courte longueur dans lequel est inséré le port. Le port est fixé au tube par collage à l'aide d'une colle type cyanoacrylate, la paroi extérieure du tube étant soudé à la paroi de l'enceinte.

Le polyéthylène utilisé pour les tubes est un matériau difficile à coller, le maintien entre le tube et le port étant donc peu efficace, ce qui engendre des problèmes de tenue du tout assemblé et par conséquent des problèmes potentiels de fuite si le collage n'est pas efficace. En particulier lors du retrait de l'aiguille il y a un risque d'arrachement du port qui se désolidarise du tube.

L'utilisation de la colle pose aussi des problèmes de toxicité et des risques de contamination de l'assemblage si le procédé de collage n'est pas effectué correctement, ce qui est particulièrement gênant si le contenu de l'enceinte doit absolument éviter toute contamination chimique et/ou particulaire,

Il existe par conséquent un besoin de développer un moyen alternatif pour fixer un port destiné à une aiguille à une enceinte pour les applications biopharmaceutiques ainsi que pour les dispositifs médicaux.

A cet effet, le port faisant l'objet de cette invention est tel qu'il comprend un corps rigide annulaire s'étendant selon une direction, ledit corps comprenant une paroi latérale entourant un canal s'étendant entre une extrémité extérieure et une extrémité intérieure, lesdites extrémités extérieure et intérieure étant opposées selon la direction, ledit corps étant ouvert auxdites extrémités intérieure et extérieure, un élément de surmoulage en élastomère thermoplastique, l'élément de surmoulage (20) comprenant un corps annulaire s'étendant selon la direction (X), ledit corps annulaire comprenant une surface intérieure et une surface extérieure, l'élément de surmoulage comprenant en outre une portion obturante formée d'un seul tenant avec l'élément de surmoulage, adaptée pour être traversée par l'aiguille selon une direction proche de la direction, l'aiguille passant par les extrémités intérieure et extérieure pour transférer un contenu entre l'intérieur et l'extérieur par l'intermédiaire de l'aiguille, l'élément de surmoulage obturant l'extrémité intérieure du canal de manière étanche en l'absence d'aiguille et après un retrait d'aiguille, l'élément de surmoulage comprenant en outre une portion d'adhésion adaptée pour la fixation du port à l'enceinte, caractérisé en ce que la surface intérieure de l'élément de surmoulage est en contact avec la paroi latérale du corps rigide sur une partie extérieure dudit corps rigide s'étendant continûment au-delà de l'extrémité intérieure du corps rigide, le corps annulaire de l'élément de surmoulage s'étendant continûment au-delà de l'extrémité intérieure du corps rigide et étant fermé par la portion obturante de sorte que la portion obturante est en regard avec l'extrémité intérieure un espace étant libre entre la portion obturante de l'élément de surmoulage et l'extrémité intérieure du corps rigide, et en ce que l'élément de surmoulage comprenant en outre une portion d'adhésion adaptée pour la fixation directe du port à l'enceinte, la portion d'adhésion étant au niveau de la surface extérieure de l'élément de surmoulage.

Dans ce cas, le port pourra être soudé directement sur une enceinte, ceci améliorant considérablement la tenue et l'étanchéité du port avec l'enceinte pour le transfert de fluide médical ou biopharmaceutique, et limitant les risques de contamination.

De plus l'aiguille traverse la portion obturante alors que celle-ci se trouve contenue dans l'enceinte, ce qui limite le trajet de l'aiguille et diminue le risque de percer l'enceinte avec la pointe de l'aiguille.

L'élastomère thermoplastique constituant l'élément de surmoulage étant à effet mémoire, à l'usage après passage de l'aiguille et son retrait, le pore ouvert dans l'élément de surmoulage par le passage de l'aiguille se referme, le produit contenu dans l'enceinte étant ainsi maintenu dans une enceinte redevenue étanche après retrait de l'aiguille, évitant tout risque de contamination via le pore créé.

Dans le cas des ports tels que décrits dans la plaquette commerciale Sartorius, les enceintes sont habituellement testées en intégrité par mise en pression avec ledit tube bouché assemblé, c'est à dire sans port. Ensuite, un port est collé pour chaque enceinte validée mais l'étanchéité avec le port collé n'est pas vérifiée. Dans le cas de l'invention l'enceinte sera testée avec le port déjà soudé, ce qui permet d'augmenter la fiabilité du produit pour son utilisateur.

Selon certains aspects, on peut en plus avoir recours à l'une et/ou l'autre des dispositions suivantes :
- le corps rigide est en polymère thermoplastique choisi parmi le polypropylène et le polyéthylène haute densité ;
- le corps rigide comprend des nervures creusées dans l'épaisseur de la paroi, une partie extérieure dudit corps s'étendant continûment depuis l'extrémité intérieure dudit corps, lesdites nervures étant de forme allongée et orientées dans une direction sensiblement parallèle à la direction X, lesdites nervures étant creusées dans la partie extérieure du corps ;
- les nervures sont soit rectilignes soit en zigzag le long de la direction X ;
- le corps rigide comprend un élément bouchon, l'élément bouchon ayant des dimensions adaptées pour obturer l'extrémité extérieure du corps ;
- l'élément bouchon est lié au corps rigide par un lien, l'élément bouchon, le corps rigide et le lien étant moulés en une seule pièce;
- le corps rigide comporte un élément additionnel formant une partie sécable, ladite partie sécable comprenant une extrémité extérieure et une extrémité intérieure, la partie sécable s'étendant selon la direction X depuis l'extrémité extérieure du corps rigide en définissant une zone de jonction dans la zone où l'extrémité extérieure du corps se confond avec l'extrémité intérieure de la partie sécable, l'extrémité extérieure de la partie sécable étant obturée ;
- le corps rigide est moulé, ledit corps étant fragilisé au niveau de la zone de jonction;
- la partie sécable comprend des ailettes adaptées pour permettre leur préhension pour désolidariser la partie sécable du corps rigide;
- lesdites ailettes sont moulées avec la partie sécable.

Selon un deuxième aspect, l'invention a pour objet un système de distribution d'un contenu par l'intermédiaire d'une aiguille entre l'intérieur d'une enceinte et l'extérieur d'une enceinte, dans le domaine biopharmaceutique et médical, ledit système comprenant un tel port et une enceinte, ladite enceinte comprenant une paroi avec une ouverture dans sa paroi, l'élément de surmoulage étant partiellement inséré dans l'enceinte par l'ouverture, la paroi de l'enceinte au niveau de son ouverture étant soudée à la portion d'adhésion de l'élément de surmoulage.

En particulier le système de distribution est tel que l'enceinte est une poche souple en film plastique pour contenus biopharmaceutiques ou pour dispositif médical et plus particulièrement une poche souple à usage unique, et plus particulièrement encore une poche souple à usage unique ayant une couche contact au fluide biopharmaceutique composée d'EVA (acétate de vinyle d'éthylène).

Selon un troisième aspect, l'invention a pour objet un procédé de fabrication d'un port selon l'invention, adapté pour être fixé à une enceinte et adapté pour être traversé par une aiguille utilisée pour le transfert d'un contenu entre l'intérieur de l'enceinte et l'extérieure à l'enceinte, dans le domaine biopharmaceutique et médical, pour lequel on fournit un corps rigide annulaire s'étendant selon une direction, ledit corps comprenant une paroi latérale entourant un canal s'étendant entre une extrémité intérieure et une extrémité extérieure, lesdites extrémités extérieure et intérieure étant opposées selon la direction, ledit corps étant ouvert auxdites extrémités intérieure et extérieure, une partie extérieure dudit corps s'étendant continûment depuis l'extrémité intérieur dudit corps, un système de moules, adapté pour permettre la fabrication d'un élément de surmoulage en élastomère thermoplastique, ledit système de moules comprenant un premier moule et un second moule,un produit de moulage, caractérisé en ce que le procédé de fabrication comprenant les étapes suivantes de placer le système de moules de façon adaptée pour permettre la fabrication d'un élément de surmoulage, tel que le premier moule enveloppe la partie extérieure dudit corps et le second moule est inséré dans le corps rigide, ledit élément de surmoulage comprenant une portion obturante adaptée pour être traversée par l'aiguille selon une direction proche de la direction, l'aiguille passant par les extrémités intérieure et extérieure pour transférer un contenu entre l'intérieur et l'extérieur par l'intermédiaire de l'aiguille, l'élément de surmoulage obturant le canal de manière étanche en l'absence d'aiguille après un retrait d'aiguille, l'élément de surmoulage comprenant en outre une portion d'adhésion adaptée pour la fixation du port à l'enceinte, de mouler ledit élément de surmoulage en versant le produit de moulage entre les premier et deuxième moule, puis de retirer le système de moules.

En particulier le procédé de fabrication d'un port via la technique de surmoulage par transfert : est tel que
- le système de moules comprend deux moules, un premier moule adapté pour envelopper la partie extérieure dudit corps et un second moule adapté pour être inséré dans le corps rigide, l'étape de moulage consistant à verser le produit de moulage entre les premier et deuxième moule ;
- le corps (10) est obtenu lors d'une étape préalable de moulage.

Selon un quatrième aspect, l'invention a pour objet un procédé de fabrication d'un système de distribution d'un contenu par l'intermédiaire d'une aiguille entre l'intérieur d'une enceinte et l'extérieur d'une enceinte, dans le domaine biopharmaceutique et médical, pour lequel on fournit un port, une enceinte, ladite enceinte comprenant une paroi avec une ouverture dans sa paroi, le procédé comprenant comme étape que l'élément de surmoulage est partiellement inséré dans l'enceinte par l'ouverture, la paroi de l'enceinte au niveau de son ouverture est soudée par soudure haute fréquence au niveau de la portion d'adhésion de l'élément de surmoulage en appliquant un champ électromagnétique à travers le corps rigide.

On décrit maintenant brièvement les figures des dessins.
La figure 1 est une vue de côté partiellement en coupe illustrant un port ;
La figure 2 est une vue de côté partiellement en coupe illustrant un système de distribution ;
La figure 3 illustre un port avec des nervures;
La figure 4 est une vue de côté illustrant un port avec un bouchon ;
La figure 5 est une vue similaire à la figure 1 pour un deuxième mode de réalisation d'un port avec une partie sécable et des ailettes ;
La figure 6 est un gros plan de la figure 5 illustrant la zone de jonction ;
Les figures 7a et 7b sont des vues schématiques illustrant diverses étapes de fabrication d'un mode de réalisation de port.

Ci-après un exposé détaillé de plusieurs modes de réalisation de l'invention assortis d'exemples et de référence aux figures.

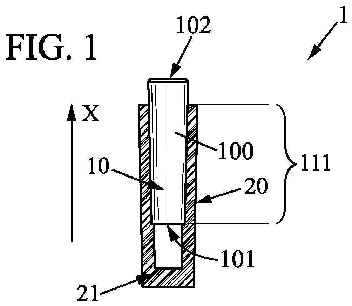

La **figure 1** illustre un port 1 comprenant un corps rigide 10 annulaire s'étendant selon une direction X, ledit corps 10 comprenant une paroi latérale 100 entourant un canal s'étendant entre une extrémité intérieure 101 et une extrémité extérieure 102, lesdites extrémités intérieure et extérieure et 101, 102 étant opposées selon la direction X, ledit corps étant ouvert auxdites extrémités intérieure et extérieure 101, 102. Dans le cas où une aiguille est reçue par le port, elle sera orientée dans une direction proche de la direction X et traversant chacune desdites extrémités 101 et 102.

Le port 1 comprend également un élément de surmoulage 20 en élastomère thermoplastique.

L'élément de surmoulage 20 comprend une portion obturante 21 adaptée pour être traversée par l'aiguille selon une direction proche de la direction X, l'aiguille passant par les extrémités intérieure et extérieure 101, 102 pour permettre le transfert d'un contenu entre l'intérieur et l'extérieur par l'intermédiaire de l'aiguille. L'élément de surmoulage obture le canal de manière étanche en l'absence d'aiguille après un retrait d'aiguille.

L'élément de surmoulage comprend en outre une portion d'adhésion adaptée pour la fixation du port à l'enceinte.Le surmoulage 20 est par exemple un corps annulaire s'étendant selon la direction X et fermé à l'une de ses extrémités par sa portion obturante 21. Le surmoulage s'étendant autour du corps 10 et comprend une surface intérieure et une surface extérieure, de sorte que la surface intérieure du surmoulage 20 est en contact avec la paroi latérale 100 du corps rigide 10, sur une partie extérieure 111 dudit corps s'étendant continûment depuis l'extrémité intérieure 101 dudit corps 10. Le corps annulaire qui constitue le surmoulage s'étend continûment au delà de l'extrémité intérieure 101 du corps 10, et est fermé par sa portion obturante 21, de sorte que sa portion obturante est en regard avec l'extrémité intérieure 101 ouverte, un espace étant libre entre la portion obturante 21 du surmoulage 20 et l'extrémité intérieure 101 du corps 10. La portion d'adhésion du surmoulage est donc au niveau de la surface extérieure du surmoulage.

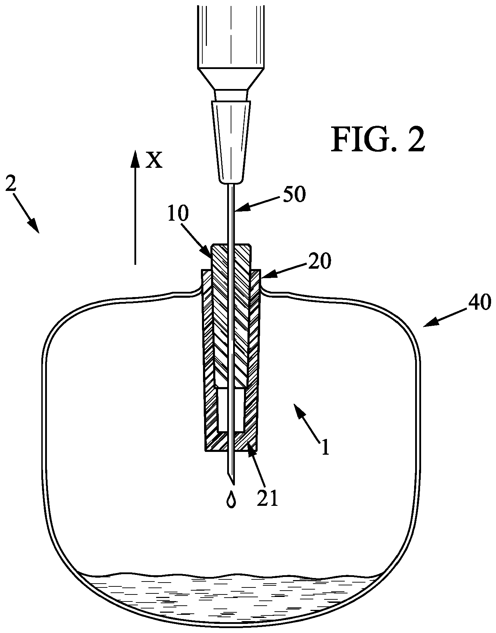

A l'usage, pour permettre le transfert d'un contenu entre l'intérieur de l'enceinte 40 et l'extérieur de l'enceinte 40, dans le domaine biopharmaceutique et médical, par l'intermédiaire d'une aiguille 50 à travers un tel port 1, ladite enceinte 40 sera soudée directement sur le port 1 traversable par l'aiguille utilisée pour le transfert, l'ensemble de l'enceinte et du port formant un système de distribution 2. Plus précisément, l'enceinte 40 pourra être par exemple une poche souple avec une paroi avec une ouverture réservée dans sa paroi Comme illustré à la **figure 2****,** l'élément de surmoulage 20 sera partiellement inséré dans la poche 40 par son ouverture et la paroi de la poche au niveau de son ouverture sera soudée au niveau de la portion d'adhésion de l'élément de surmoulage 20. Dans l'exemple représenté, le port 1 est représenté assemblé sur la partie intérieure de la paroi de la poche, de sorte que l'extrémité intérieure 101 du corps 10 est une extrémité intérieure à la poche, et que l'extrémité extérieure 102 est une extrémité extérieure à la poche. En variante, le port pourra être assemblé sur la poche à proximité de la portion obturante 21 du port de sorte que l'extrémité intérieure 101 du corps 10 est une extrémité extérieure à la poche, et que l'extrémité extérieure 102 est une extrémité extérieure à la poche.

Ainsi, dans le cas où le port 1 recevra une aiguille 50, l'aiguille 50 traversera la partie de l'élément de surmoulage 20 enveloppant l'extrémité intérieure 101, dite zone septum 21, ou portion obturante, de sorte que le contenu de l'aiguille (ou d'une seringue) pourra être transféré dans la poche par l'intermédiaire de l'aiguille. Le port 1 pourra notamment être utilisé pour qu'y soit insérée l'aiguille d'une seringue, pour ajouter des produits via une seringue à un mélange initialement contenu dans la poche 40. On pourra également utiliser le port 1 pour qu'y soit inséré une aiguille d'une seringue à des fins de prélèvement du contenu de la poche.

Plus précisément, l'élément de surmoulage 20 est en élastomère thermoplastique. Le choix d'un tel matériau, à effet mémoire, permettra à l'usage qu'après passage de l'aiguille à travers la portion obturante et son retrait, le pore ouvert dans l'élément de surmoulage 20 par le passage de l'aiguille se referme, pour éviter tout risque de contamination notamment.

De plus le choix d'un élastomère thermoplastique pour l'élément de surmoulage 20 permettra une soudure facilitée d'une poche 40 en film plastique sur le surmoulage 20.

On pourra notamment utiliser une poche en film EVA (acétate de vinyle d'éthylène) adaptée pour contenir des dérivés sanguins et des bio-médicaments. Un tel matériau pour la poche, facilement soudé à l'élément de surmoulage 20, confère une grande souplesse à la poche 40. Une telle poche pourra de plus être stérilisée par rayonnement gamma ou ETO (oxyde d'éthylène) sur demande pour permettre un remplissage aseptique, par exemple pour une stérilisation de l'ensemble du port et de la poche après leur assemblage.

Le corps rigide 10 est par exemple formé dans un polymère thermoplastique. Le corps 10 peut notamment être du polypropylène. En variante le corps 10 pourra être en polyéthylène haute densité.

Nous allons exposer ci-dessous des modes de réalisation pouvant être pris en combinaison.

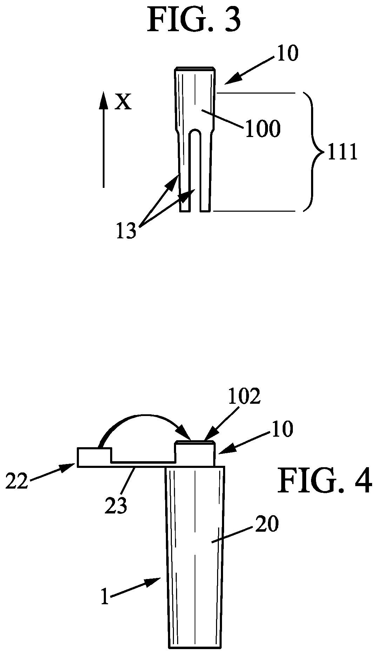

Dans un mode de réalisation illustré à la **figure 3****,** la partie extérieure 111 du corps rigide 10 pourra comprendre des nervures 13 creusées dans l'épaisseur de la paroi 100, lesdites nervures 13 étant de forme allongée et orientées selon une direction sensiblement parallèle à la direction X, lesdites nervures 13 étant creusées dans la partie extérieure 111 du corps rigide 10. Lesdites nervures 13 seront par exemple creusées depuis la surface extérieure de la paroi 100 du corps rigide 10, dans l'épaisseur de la paroi 100. Avantageusement les nervures 13 pourront déboucher à travers la paroi 100 sur toute leur longueur c'est-à-dire que l'épaisseur de la paroi pourra être totalement creusée au niveau des nervures 13. En variante, les nervures 13 pourront être en zigzag par rapport à leur direction d'extension générale précédemment présentée.

De telles nervures 13 permettent notamment une meilleure adhérence de l'élément de surmoulage 20 à la surface du corps 10.

Dans un mode de réalisation illustré à la **figure 4****,** le corps rigide 10 comprend un élément bouchon 22, l'élément bouchon 22 ayant des dimensions adaptées pour obturer l'extrémité extérieure du corps 10. L'élément bouchon 22 est par exemple lié au corps rigide par un lien 23, l'élément bouchon 22, le corps rigide 10 et le lien 23 étant moulés en une seule pièce. Le moulage est tel que le lien 23 s'étend radialement depuis la paroi latérale 100 en dessous de l'extrémité extérieure 102.

En variante, l'élément de surmoulage 20 pourra comprendre un élément bouchon 22, ledit élément bouchon étant solidaire de la portion obturante de l'élément de surmoulage, l'élément bouchon 22 ayant des dimensions adaptées pour obturer l'extrémité extérieure 102 du corps 10. L'élément bouchon 22, l'élément de surmoulage 20 et un lien 23 reliant l'élément bouchon 22 avec la portion obturante 21 sont par exemple moulés en une seule pièce. L'élément bouchon 22 a par exemple des dimensions adaptées pour être inséré dans le corps rigide 10 par l'extrémité extérieure 102, il a par exemple une forme tronconique, et/ou est compressible, ce qui lui permet d'être inséré en force dans l'extrémité extérieure 102 du corps 10 et de le fermer. En variante, l'élément bouchon 22 a des dimensions adaptées pour s'encastrer autour du corps rigide 10 au niveau de son extrémité extérieure 102, il a par exemple une section circulaire de diamètre supérieur au diamètre de la section circulaire du corps rigide 10, de sorte que l'élément bouchon 22 englobe une partie de la paroi 100 du corps rigide 10 au niveau de son extrémité extérieure 102.

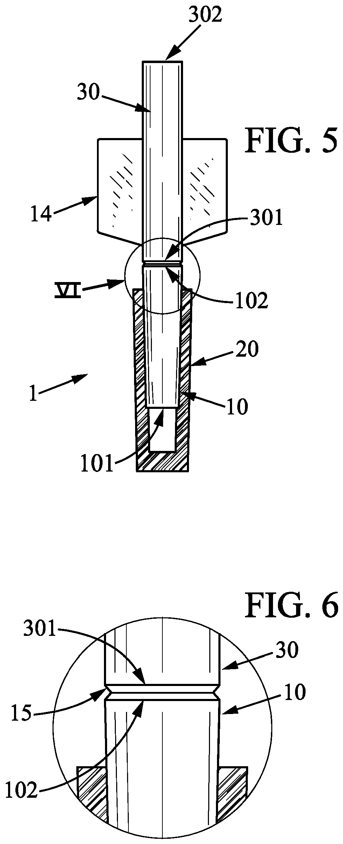

Dans un mode de réalisation illustré à la **figure 5****,** le corps rigide 10 comporte un élément additionnel formant une partie sécable 30, ladite partie sécable 30 comprenant une extrémité extérieure 302 et une extrémité intérieure 301, la partie sécable 30 s'étendant selon la direction (X) en surmontant le corps rigide 10 depuis l'extrémité extérieure 102 du corps rigide 10 en définissant une zone de jonction 15 dans la zone où l'extrémité extérieure du corps 102 se confond avec l'extrémité intérieure de la partie sécable 301. L'extrémité extérieure de la partie sécable 302 est obturée. Elle peut notamment être obturée par soudure par ultrason.

Le corps rigide 10 est par exemple moulé avec sa partie sécable 30, l'ensemble du corps rigide 10 et de sa partie sécable 30 étant fragilisé au niveau de la zone de jonction 15 comme illustré à la **figure 6****.** La zone de jonction 15 entre le corps rigide 10 et sa partie sécable 30 est ainsi fragilisée, ce qui permettra une rupture facilitée au niveau de cette zone de jonction par application d'une force dans une direction transverse à la direction (X).

Une telle partie sécable 30 permet notamment d'éviter tout risque de contamination, impureté ou poussière du port 1 avant sa première utilisation, l'extrémité extérieure 302 de la partie sécable étant fermée. L'utilisateur pourra désolidariser la partie sécable 30 du reste du corps rigide 10 avant sa première utilisation, la partie sécable 30 jouant le rôle de témoin d'inviolabilité, c'est-à-dire que, tant qu'elle n'aura pas été retirée, on pourra assurer que le port 1 n'aura pas été utilisé. Les ports comprenant une partie sécable 30 pourront également être plus facilement transportés sans besoin d'un emballage particulièrement protecteur.

Dans un mode avantageux de réalisation, la partie sécable 30 pourra comprendre des ailettes 14, comme illustrées à la **figure 5****,** adaptées pour permettre leur préhension pour désolidariser la parte sécable 30 du corps rigide 10. Lesdites ailettes 14 pourront par exemple être moulées avec la partie sécable 30.

Le procédé de fabrication d'un tel port 1 comprendra les étapes de fourniture d'un corps rigide 10 tel que décrit précédemment, ainsi que d'un système de moules 3, adapté pour permettre la fabrication d'un élément de surmoulage 20 en élastomère thermoplastique, ainsi que d'un produit de moulage 33.

La fabrication consistera à placer le système de moules 3 de façon adaptée pour permettre la fabrication d'un élément de surmoulage 20, enveloppant la partie extérieure 111 dudit corps et la surface d'extrémité intérieure dudit corps 101, à mouler ledit élément de surmoulage 20, puis à retirer le système de moules 3.

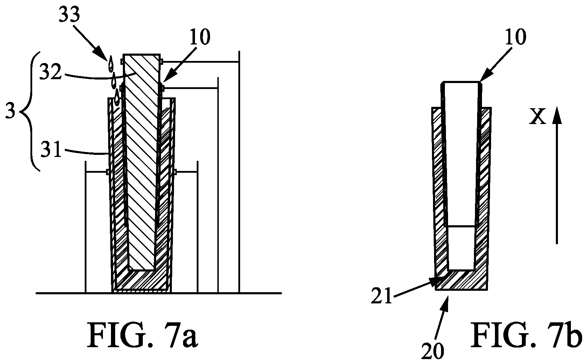

Dans un mode de réalisation, le système de moules 3 pourra comprend deux moules, un premier moule 31 adapté pour envelopper la partie extérieure 111 dudit corps 10 et un second moule 32 adapté pour être inséré dans le corps rigide 10. Le premier moule 31 pourra par exemple être un cylindre d'axe (X) et fermé à l'une de ses extrémités dans lequel pourra être inséré le corps 10, et le second moule 32 pourra être un cylindre d'axe (X) et de diamètre très légèrement inférieur au diamètre du corps annulaire 10. Comme illustré à la **figure 7a****,** le corps 10 pourra être inséré au moins partiellement dans le premier moule 31 et le second moule 32 pourra traverser le corps 10 de part en part en débouchant à traverse chacune des extrémités du corps 10. La paroi du corps 10 sera comprise entre la surface latérale du premier moule 31 et la surface latérale du second moule 32, puis le produit de moulage 33 pourra être versé entre les premier et deuxième moule 31, 32. Une petite quantité de produit de moulage pourra en particulier s'insérer entre la surface latérale du second moule 32 et la paroi du corps 10. Comme illustré à la **figure 7b****,** le système de moules 3 sera ensuite retiré, libérant le port 1.

L'élément de surmoulage 20 sera partiellement inséré dans l'enceinte 40 par l'ouverture, puis la paroi de l'enceinte au niveau de son ouverture sera soudée par un procédé de soudure froide, type soudure haute fréquence, à la portion d'adhésion de l'élément de surmoulage 20, en appliquant un champ électromagnétique à travers le corps rigide 10, la paroi de l'enceinte au niveau de son ouverture et l'élément de surmoulage 20, qui vont ainsi se souder par transfert de l'énergie du champ électromagnétique.

Des nervures 13 creusées dans le corps rigide 10 comme décrit précédemment permettront notamment d'améliorer la soudure de la poche 40 à la surface de l'élément de surmoulage 20. En effet les nervures aideront à une meilleure diffusion du courant dans l'élément de surmoulage 20 ainsi rendu avantageusement malléable par effet de chauffage pour améliorer la soudure.

Dans le cas d'un port 1 comprenant une partie sécable 30, la partie sécable sera préalablement désolidarisée du corps rigide 10 avant insertion de l'aiguille 50 dans l'extrémité extérieure 102 du corps rigide 10.

La stérilité du port 1 pourra être garantie avant sa mise en oeuvre dans le système de distribution 2, par une stérilisation aux rayons gamma par exemple.

Dans le cas d'une poche EVA, l'ensemble pourra donc être stérilisé facilement.

On pourra mettre en oeuvre un système de distribution 2 du contenu entre l'extérieur et l'intérieur d'une enceinte 40 par l'intermédiaire d'une aiguille 50, en insérant une aiguille 50 dans un port 1 préalablement assemblé à ladite enceinte 40.

Après utilisation du port 1 du système de distribution 2, l'aiguille 50 sera retirée, puis le bouchon 22 permettra de fermer la chambre formée par l'enceinte au niveau du port 1, pour assurer une meilleure étanchéité.

La paroi latérale 100 du corps rigide 10 peut en variante présenter un épaulement situé sous l'extrémité extérieure 102 et au-dessus de l'attache du lien 23 reliant le bouchon 22, le lien 23 s'étend radialement depuis la paroi latérale 100. L'intérieur du bouchon 22 possède par exemple une gorge pour y emboiter l'épaulement. Cela constitue une fermeture inviolable ou difficilement ré-ouvrable sans forcer au risque de détériorer le bouchon 22.

## Revendications

1. Système de distribution (2) d'un contenu par l'intermédiaire d'une aiguille entre l'intérieur d'une enceinte et l'extérieur d'une enceinte, ledit système comprenant un port (1) et une enceinte (40)
le port (1) étant adapté pour être fixé à une enceinte et adapté pour être traversé par une aiguille utilisée pour le transfert d'un contenu entre l'intérieur de l'enceinte et l'extérieure de l'enceinte, ledit port comprenant :
- un corps rigide (10) annulaire s'étendant selon une direction (X), ledit corps (10) comprenant une paroi latérale (100) entourant un canal s'étendant entre une extrémité intérieure (101) et une extrémité extérieure (102), lesdites extrémités intérieure et extérieure (101, 102) étant opposées selon la direction (X), ledit corps étant ouvert auxdites extrémités intérieure et extérieure (101, 102),
- un élément de surmoulage (20) en élastomère thermoplastique, l'élément de surmoulage (20) comprenant un corps annulaire s'étendant selon la direction (X), ledit corps annulaire comprenant une surface intérieure et une surface extérieure, l'élément de surmoulage (20) comprenant en outre une portion obturante (21) formée d'un seul tenant avec l'élément de surmoulage, adaptée pour être traversée par l'aiguille selon une direction proche de la direction (X), l'aiguille passant par les extrémités intérieure et extérieure (101, 102) pour transférer un contenu entre l'intérieur et l'extérieur par l'intermédiaire de l'aiguille, l'élément de surmoulage obturant l'extrémité intérieure (101) du canal de manière étanche en l'absence d'aiguille après un retrait d'aiguille,
**Caractérisé en ce que** la surface intérieure de l'élément de surmoulage (20) est en contact avec la paroi latérale du corps rigide (10) sur une partie extérieure (111) dudit corps rigide (10) s'étendant continûment au-delà de l'extrémité intérieure du corps rigide (10), le corps annulaire de l'élément de surmoulage (20) s'étendant continûment au-delà de l'extrémité intérieure (101) du corps rigide (10) et étant fermé par la portion obturante (21) de sorte que la portion obturante (21) est en regard avec l'extrémité intérieure (101) un espace étant libre entre la portion obturante (21) de l'élément de surmoulage (20) et l'extrémité intérieure (101) du corps rigide,
**en ce que** l'élément de surmoulage comprenant en outre une portion d'adhésion adaptée pour la fixation directe du port à l'enceinte, la portion d'adhésion étant au niveau de la surface extérieure de l'élément de surmoulage (20),
ladite enceinte (40) comprenant une paroi avec une ouverture dans sa paroi, l'élément de surmoulage (20) étant partiellement inséré dans l'enceinte (40) par l'ouverture, la paroi de l'enceinte au niveau de son ouverture étant apte à être soudée à la portion d'adhésion de l'élément de surmoulage (20).

2. Système de distribution (2) selon la revendication 1 dans lequel le corps rigide (10) du port est en polymère thermoplastique choisi parmi le polypropylène et le polyéthylène haute densité.

3. Système de distribution (2) selon l'une quelconque des revendications 1 à 2 dans lequel le corps rigide (10) du port comprend des nervures (13) creusées dans l'épaisseur de la paroi (100), une partie extérieure (111) dudit corps s'étendant continûment depuis l'extrémité intérieure (101) dudit corps (10), lesdites nervures (13) étant de forme allongée et orientées dans une direction sensiblement parallèle à la direction (X), lesdites nervures étant creusées dans la partie extérieure (111) du corps (10).

4. Système de distribution (2) selon la revendication 3 dans lequel les nervures sont soit rectilignes soit en zigzag le long de la direction (X).

5. Système de distribution (2) selon l'une quelconque des revendications 1 à 4 dans lequel le corps rigide (10) du port comprend un élément bouchon (22), l'élément bouchon (22) ayant des dimensions adaptées pour obturer l'extrémité extérieure (102) du corps (10).

6. Système de distribution (2) selon la revendication 5 dans lequel l'élément bouchon (22) est lié au corps rigide (10) par un lien (23), l'élément bouchon (22), le corps rigide (10) et le lien (23) étant moulés en une seule pièce.

7. Système de distribution (2) selon l'une quelconque des revendications 1 à 6 dans lequel le corps rigide (10) du port comporte un élément additionnel formant une partie sécable (30), ladite partie sécable (30) comprenant une extrémité extérieure (302) et une extrémité intérieure (301), la partie sécable (30) s'étendant selon la direction (X) depuis l'extrémité extérieure (102) du corps rigide (10) en définissant une zone de jonction (15) dans la zone où l'extrémité extérieure du corps (102) se confond avec l'extrémité intérieure de la partie sécable (301), l'extrémité extérieure de la partie sécable (302) étant obturée.

8. Système de distribution (2) selon la revendication 7 dans lequel le corps rigide (10) est moulé, ledit corps étant fragilisé au niveau de la zone de jonction (15).

9. Système de distribution (2) selon l'une quelconque des revendications 7 et 8 dans lequel la partie sécable (30) comprend des ailettes (14) adaptées pour permettre leur préhension pour désolidariser la partie sécable (30) du corps rigide (10).

10. Système de distribution (2) selon la revendication 9 dans lequel lesdites ailettes (14) sont moulées avec la partie sécable (30).

11. Système de distribution selon l'une quelconque des revendications 1 à 10 pour lequel l'enceinte (40) est une poche souple en film plastique pour contenus biopharmaceutiques.

12. Procédé de fabrication d'un port tel que compris dans un système de distribution selon l'une des revendications 1 à 11, ledit port étant adapté pour être fixé à l'enceinte du système de distribution et adapté pour être traversé par une aiguille utilisée pour le transfert d'un contenu entre l'intérieur de l'enceinte et l'extérieure de l'enceinte, pour lequel on fournit :
- un corps rigide (10) annulaire s'étendant selon une direction (X), ledit corps (10) comprenant une paroi latérale (100) entourant un canal s'étendant entre une extrémité intérieure (101) et une extrémité extérieure (102), lesdites extrémités intérieure et extérieure (101, 102) étant opposées selon la direction (X), ledit corps étant ouvert auxdites extrémités intérieure et extérieure (101, 102), une partie extérieure (111) dudit corps s'étendant continûment depuis l'extrémité intérieure (101) dudit corps (10),
- un système de moules (3), adapté pour permettre la fabrication d'un élément de surmoulage (20) en élastomère thermoplastique, ledit système de moules (3) comprenant un premier moule (31) et un second moule (32),
- un produit de moulage (33),
**Caractérisé en ce que** le procédé de fabrication comprend les étapes suivantes :
- placer le système de moules (3) de façon adaptée pour permettre la fabrication d'un élément de surmoulage (20), tel que le premier moule (31) enveloppe la partie extérieure (111) dudit corps (10) et le second moule (32) est inséré dans le corps rigide (10), ledit élément de surmoulage comprenant une portion obturante (21) adaptée pour être traversée par l'aiguille selon une direction proche de la direction (X), l'aiguille passant par les extrémités intérieure et extérieure (101, 102) pour transférer un contenu entre l'intérieur et l'extérieur par l'intermédiaire de l'aiguille, l'élément de surmoulage obturant le canal de manière étanche en l'absence d'aiguille après un retrait d'aiguille, l'élément de surmoulage comprenant en outre une portion d'adhésion adaptée pour la fixation du port à l'enceinte, l'élément de surmoulage (20) comprenant un corps annulaire s'étendant selon la direction (X), ledit corps annulaire comprenant une surface intérieure et une surface extérieure, la surface intérieure de l'élément de surmoulage (20) étant en contact avec la paroi latérale du corps rigide (10) sur une partie extérieure (111) dudit corps rigide (10) s'étendant continûment au-delà de l'extrémité intérieure du corps rigide (10), le corps annulaire de l'élément de surmoulage (20) s'étendant continûment au-delà de l'extrémité intérieure (101) du corps rigide (10) et étant fermé par la portion obturante (21) de sorte que la portion obturante (21) est en regard avec l'extrémité intérieure (101) un espace étant libre entre la portion obturante (21) de l'élément de surmoulage (20) et l'extrémité intérieure (101) du corps rigide, la portion d'adhésion étant au niveau de la surface extérieure de l'élément de surmoulage (20)
- mouler ledit élément de surmoulage (20) en versant le produit de moulage (33) entre les premier et deuxième moule (31, 32),
- retirer le système de moules (3).

13. Procédé de fabrication d'un port selon la revendication 12 dans lequel on obtient le corps (10) lors d'une étape préalable de moulage.

14. Procédé de fabrication d'un système de distribution (2) d'un contenu par l'intermédiaire d'une aiguille entre l'intérieur d'une enceinte et l'extérieur d'une enceinte, pour lequel on fournit :
- un port (1) et une enceinte (40), tels que décrits dans l'une quelconque des revendications 1 à 11,
le procédé comprenant comme étape:
- l'élément de surmoulage (20) est partiellement inséré dans l'enceinte (40) par l'ouverture,
- la paroi de l'enceinte au niveau de son ouverture est soudée à la portion d'adhésion de l'élément de surmoulage (20) en appliquant un champ électromagnétique haute fréquence à travers le corps rigide (10).

## Patentansprüche

1. System (2) zur Abgabe eines Inhalts über eine Nadel zwischen dem Inneren eines Behälters und dem Äußeren eines Behälters, wobei das System einen Anschluss (1) und einen Behälter (40) umfasst,
wobei der Anschluss (1) angepasst ist, um an einem Behälter befestigt zu werden, und angepasst ist, um durch eine Nadel durchquert zu werden, die für die Übertragung eines Inhaltes zwischen dem Inneren des Behälters und dem Äußeren des Behälters verwendet wird, wobei der Anschluss Folgendes umfasst:
- einen ringförmigen starren Körper (10), der sich entlang einer Richtung (X) erstreckt, wobei der Körper (10) eine seitliche Wand (100) umfasst, die einen Kanal umgibt, der sich zwischen einem inneren Ende (101) und einem äußeren Ende (102) erstreckt, wobei das innere und das äußere Ende (101, 102) entlang der Richtung (X) entgegengesetzt sind, wobei der Körper an dem inneren und dem äußeren Ende (101, 102) offen ist,
- ein Überformungselement (20) aus thermoplastischem Elastomer, wobei das Überformungselement (20) einen ringförmigen Körper umfasst, der sich entlang der Richtung (X) erstreckt, wobei der ringförmige Körper eine innere Oberfläche und eine äußere Oberfläche umfasst, wobei das Überformungselement (20) ferner einen verschließenden Abschnitt (21) umfasst, der einteilig mit dem Überformungselement gebildet ist und angepasst ist, um durch die Nadel entlang einer Richtung nahe der Richtung (X) durchquert zu werden, wobei die Nadel durch das innere und das äußere Ende (101, 102) verläuft, um einen Inhalt zwischen dem Inneren und dem Äußeren über die Nadel zu übertragen, wobei das Überformungselement bei Nichtvorhandensein einer Nadel nach einem Entfernen einer Nadel das innere Ende (101) des Kanals auf dichte Weise verschließt,
**dadurch gekennzeichnet, dass** die innere Oberfläche des Überformungselements (20) mit der seitlichen Wand des starren Körpers (10) über einen äußeren Teil (111) des starren Körpers (10) in Kontakt ist, der sich ununterbrochen über das innere Ende des starren Körpers (10) hinaus erstreckt, wobei der ringförmige Körper des Überformungselements (20) sich ununterbrochen über das innere Ende (101) des starren Körpers (10) hinaus erstreckt und derart durch den verschließenden Abschnitt (21) verschlossen ist, dass der verschließende Abschnitt (21) sich dem inneren Ende (101) gegenüberliegend befindet, wobei ein Raum zwischen dem verschließenden Abschnitt (21) des Überformungselements (20) und dem inneren Ende (101) des starren Körpers frei ist,
und dadurch, dass das Überformungselement ferner einen Haftabschnitt umfasst, der für die direkte Befestigung des Anschlusses am Behälter angepasst ist, wobei der Haftabschnitt sich im Bereich der äußeren Oberfläche des Überformungselements (20) befindet,
wobei der Behälter (40) eine Wand mit einer Öffnung in seiner Wand umfasst, wobei das Überformungselement (20) teilweise über die Öffnung in den Behälter (40) eingesetzt ist, wobei die Wand des Behälters im Bereich seiner Öffnung geeignet ist, an den Haftabschnitt des Überformungselements (20) geschweißt zu werden.

2. Abgabesystem (2) nach Anspruch 1, wobei der starre Körper (10) des Anschlusses aus thermoplastischem Polymer besteht, das aus Polypropylen und Polyethylen mit hoher Dichte ausgewählt ist.

3. Abgabesystem (2) nach einem der Ansprüche 1 bis 2, wobei der starre Körper (10) des Anschlusses Rippen (13) umfasst, die in der Dicke der Wand (100) vertieft sind, wobei ein äußerer Teil (111) des Körpers sich ununterbrochen ausgehend von dem inneren Ende (101) des Körpers (10) erstreckt, wobei die Rippen (13) eine längliche Form aufweisen und in einer Richtung im Wesentlichen parallel zur Richtung (X) ausgerichtet sind, wobei die Rippen im äußeren Teil (111) des Körpers (10) vertieft sind.

4. Abgabesystem (2) nach Anspruch 3, wobei die Rippen entlang der Richtung (X) entweder geradlinig oder zickzackförmig sind.

5. Abgabesystem (2) nach einem der Ansprüche 1 bis 4, wobei der starre Körper (10) des Anschlusses ein Pfropfenelement (22) umfasst, wobei das Pfropfenelement (22) Abmessungen aufweist, die angepasst sind, um das äußere Ende (102) des Körpers (10) zu verschließen.

6. Abgabesystem (2) nach Anspruch 5, wobei das Pfropfenelement (22) durch eine Verbindung (23) mit dem starren Körper (10) verbunden ist, wobei das Pfropfenelement (22), der starre Körper (10) und die Verbindung (23) einteilig geformt sind.

7. Abgabesystem (2) nach einem der Ansprüche 1 bis 6, wobei der starre Körper (10) des Anschlusses ein zusätzliches Element umfasst, das einen teilbaren Teil (30) bildet, wobei der teilbare Teil (30) ein äußeres Ende (302) und ein inneres Ende (301) umfasst, wobei der teilbare Teil (30) sich entlang der Richtung (X) ausgehend vom äußeren Ende (102) des starren Körpers (10) erstreckt und dabei eine Verbindungszone (15) in der Zone definiert, wo das äußere Ende des Körpers (102) mit dem inneren Ende des teilbaren Teils (301) zusammenfällt, wobei das äußere Ende des teilbaren Teils (302) verschlossen ist.

8. Abgabesystem (2) nach Anspruch 7, wobei der starre Körper (10) geformt ist, wobei der Körper im Bereich der Verbindungszone (15) geschwächt ist.

9. Abgabesystem (2) nach einem der Ansprüche 7 und 8, wobei der teilbare Teil (30) Flügel (14) umfasst, die angepasst sind, um ihr Greifen zum Trennen des teilbaren Teils (30) von dem starren Körper (10) zu ermöglichen.

10. Abgabesystem (2) nach Anspruch 9, wobei die Flügel (14) mit dem teilbaren Teil (30) geformt sind.

11. Abgabesystem nach einem der Ansprüche 1 bis 10, für das der Behälter (40) ein biegsamer Beutel aus Kunststofffilm für biopharmazeutische Inhalte ist.

12. Verfahren zur Herstellung eines Anschlusses, wie in einem Abgabesystem nach einem der Ansprüche 1 bis 11 enthalten, wobei der Anschluss angepasst ist, um an dem Behälter des Abgabesystems befestigt zu werden, und angepasst ist, um von einer Nadel durchquert zu werden, die für die Übertragung eines Inhalts zwischen dem Inneren des Behälters und dem Äußeren des Behälters verwendet wird, für das Folgendes bereitgestellt wird:
- ein ringförmiger starrer Körper (10), der sich entlang einer Richtung (X) erstreckt, wobei der Körper (10) eine seitliche Wand (100) umfasst, die einen Kanal umgibt, der sich zwischen einem inneren Ende (101) und einem äußeren Ende (102) erstreckt, wobei das innere und das äußere Ende (101, 102) entlang der Richtung (X) entgegengesetzt sind, wobei der Körper an dem inneren und dem äußeren Ende (101, 102) offen ist, wobei ein äußerer Teil (111) des Körpers sich ununterbrochen ausgehend vom inneren Ende (101) des Körpers (10) erstreckt;
- ein System von Formen (3), das angepasst ist, um die Herstellung eines Überformungselements (20) aus thermoplastischem Elastomer zu ermöglichen, wobei das System von Formen (3) eine erste Form (31) und eine zweite Form (32) umfasst,
- ein Formgussprodukt (33),
**dadurch gekennzeichnet, dass** das Herstellungsverfahren die folgenden Schritte umfasst:
- Platzieren des Systems von Formen (3) auf angepasste Art, um die Herstellung eines Überformungselements (20) zu ermöglichen, derart dass die erste Form (31) den äußeren Teil (111) des Körpers (10) umgibt und die zweite Form (32) in den starren Körper (10) eingesetzt wird, wobei das Überformungselement einen verschließenden Abschnitt (21) umfasst, der angepasst ist, um durch die Nadel entlang einer Richtung nahe der Richtung (X) durchquert zu werden, wobei die Nadel durch das innere und das äußere Ende (101, 102) verläuft, um einen Inhalt zwischen dem Inneren und dem Äußeren über die Nadel zu übertragen, wobei das Überformungselement den Kanal bei Nichtvorhandensein einer Nadel nach einem Entfernen der Nadel auf dichte Weise verschließt, wobei das Überformungselement ferner einen Haftabschnitt umfasst, der für die Befestigung des Anschlusses an dem Behälter angepasst ist, wobei das Überformungselement (20) einen ringförmigen Körper umfasst, der sich entlang der Richtung (X) erstreckt, wobei der ringförmige Körper eine innere Oberfläche und eine äußere Oberfläche umfasst, wobei die innere Oberfläche des Überformungselements (20) mit der seitlichen Wand des starren Körpers (10) über einen äußeren Teil (111) des starren Körpers (10) in Kontakt ist, der sich ununterbrochen über das innere Ende des starren Körpers (10) hinaus erstreckt, wobei der ringförmige Körper des Überformungselements (20) sich ununterbrochen über das innere Ende (101) des starren Körpers (10) hinaus erstreckt und derart durch den verschließenden Abschnitt (21) verschlossen ist, dass der verschließende Abschnitt (21) sich dem inneren Ende (101) gegenüberliegend befindet, wobei ein Raum zwischen dem verschließenden Abschnitt (21) des Überformungselements (20) und dem inneren Ende (101) des starren Körpers frei ist, wobei der Haftabschnitt sich im Bereich der äußeren Oberfläche des Überformungselements (20) befindet,
- Formen des Überformungselements (20) durch Gießen des Formprodukts (33) zwischen die erste und die zweite Form (31, 32),
- Entfernen des Systems von Formen (3).

13. Verfahren zur Herstellung eines Anschlusses nach Anspruch 12, wobei der Körper (10) bei einem vorhergehenden Formschritt erhalten wird.

14. Verfahren zur Herstellung eines Systems (2) zur Abgabe eines Inhalts über eine Nadel zwischen dem Inneren eines Behälters und dem Äußeren eines Behälters, für das Folgendes bereitgestellt wird:
- ein Anschluss (1) und ein Behälter (40) nach einem der Ansprüche 1 bis 11,
wobei das Verfahren als Schritte Folgendes umfasst:
- das Überformungselement (20) wird teilweise über die Öffnung (40) in den Behälter eingeführt,
- die Wand des Behälters im Bereich seiner Öffnung wird an den Haftabschnitt des Überformungselements (20) durch Anlegen eines hochfrequenten elektromagnetischen Feldes durch den starren Körper (10) geschweißt.

## Claims

1. Dispensing system (2) for dispensing content between the inside of a container and the outside of a container by means of a needle, said system comprising a port (1) and a container (40),
the port (1) being suitable for attachment to a container and suitable for being traversed by a needle used for the transfer of content between the inside of the container and the outside of the container, said port comprising:
- a rigid ring-shaped body (10) extending in a direction (X), said body (10) comprising a side wall (100) surrounding a channel extending between an inner end (101) and an outer end (102), said inner and outer ends (101, 102) being at opposite ends along the direction (X), said body being open at said inner and outer ends (101, 102),
- an overmolding member (20) made of thermoplastic elastomer, the overmolding member (20) comprising a ring-shaped body extending along the direction (X), said ring-shaped body comprising an inner surface and an outer surface, the overmolding member (20) further comprising a sealing portion (21) integrally formed with the overmolding member, suitable for being traversed by the needle in a direction close to the direction (X), the needle passing through the inner and outer ends (101, 102) in order to transfer content between the inside and the outside by means of the needle, the overmolding member sealing the inner end (101) of the channel in a fluidtight manner in the absence of a needle after withdrawal of the needle,
**characterized in that** the inner surface of the overmolding member (20) is in contact with the side wall of the rigid body (10) on an outer portion (111) of said rigid body (10) extending continuously beyond the inner end of the rigid body (10), the ring-shaped body of the overmolding member (20) extending continuously beyond the inner end (101) of the rigid body (10) and being closed by the sealing portion (21) such that the sealing portion (21) is facing the inner end (101), a space being free between the sealing portion (21) of the overmolding element (20) and the inner end (101) of the rigid body,
**in that** the overmolding member further comprises an adhesion portion suitable for direct attachment of the port to the container, the adhesion portion being at the level of the outer surface of the overmolding member (20),
said container (40) comprising a wall with an opening in its wall, the overmolding member (20) being partially inserted into the container (40) through the opening, the wall of the container at its opening being able to be welded to the adhesion portion of the overmolding member (20).

2. Dispensing system (2) according to claim 1, wherein the rigid body (10) of the port is made of a thermoplastic polymer selected among polypropylene and high-density polyethylene.

3. Dispensing system (2) according to any of claims 1 to 2, wherein the rigid body (10) of the port comprises ribs (13) formed in the thickness of the wall (100), an outer portion (111) of said body extending continuously from the inner end (101) of said body (10), said ribs (13) being of elongate shape and oriented in a direction substantially parallel to the direction (X), said ribs being formed in the outer portion (111) of the body (10).

4. Dispensing system (2) according to claim 3, wherein the ribs either extend in a straight line or zigzag along the direction (X).

5. Dispensing system (2) according to any of claims 1 to 4, wherein the rigid body (10) of the port comprises a cap member (22), the cap member (22) having dimensions suitable for sealing the outer end (102) of the body (10).

6. Dispensing system (2) according to claim 5, wherein the cap member (22) is connected to the rigid body (10) by a connector (23), the cap member (22), the rigid body (10), and the connector (23) being molded as one piece.

7. Dispensing system (2) according to any of claims 1 to 6, wherein the rigid body (10) of the port comprises an additional element forming a breakable portion (30), said breakable portion (30) comprising an outer end (302) and an inner end (301), the breakable portion (30) extending in the direction (X) from the outer end (102) of the rigid body (10) by defining a connection region (15) in the region where the outer end of the body (102) meets the inner end of the breakable portion (301), the outer end of the breakable portion (302) being sealed.

8. Dispensing system (2) according to claim 7, wherein the rigid body (10) is molded, said body being weakened at the connection region (15).

9. Dispensing system (2) according to any of claims 7 and 8, wherein the breakable portion (30) comprises fins (14) suitable for gripping in order to detach the breakable portion (30) from the rigid body (10).

10. Dispensing system (2) according to claim 9, wherein said fins (14) are molded with the breakable portion (30).

11. Dispensing system according to any of claims 1 to 10, wherein the container (40) is a flexible pouch of plastic film for biopharmaceutical contents.

12. Method for manufacturing a port as comprised in a distribution system according to one of claims 1 to 11, said port being suitable for attachment to a container and suitable for being traversed by a needle used to transfer content between the inside of the container and the outside of the container, wherein there is provided:
- a rigid ring-shaped body (10) extending in a direction (X), said body (10) comprising a side wall (100) surrounding a channel extending between an inner end (101) and an outer end (102), said inner and outer ends (101, 102) being at opposite ends along the direction (X), said body being open at said inner and outer ends (101, 102), an outer portion (111) of said body extending continuously from the inner end (101) of said body (10),
- a system of molds (3), suitable for the manufacture of an overmolding member (20) of thermoplastic elastomer, said system of molds (3) comprising a first mold (31) and a second mold (32),
- a molding product (33),
**characterized in that** the manufacturing method comprises the following steps of:
- suitably placing the system of molds (3) so as to enable the manufacture of an overmolding member (20), such that the first mold (31) envelopes the outer part (111) of said body (10) and the second mold (32) is inserted into the rigid body (10), said overmolding member comprising a sealing portion (21) suitable for being traversed by the needle in a direction close to the direction (X), the needle passing through the inner and outer ends (101, 102) in order to transfer content between the inside and the outside by means of the needle, the overmolding member sealing the channel in a fluidtight manner in the absence of a needle after withdrawal of the needle, the overmolding member further comprising an adhesion portion suitable for attaching the port to the container, the overmolding member (20) comprising a ring-shaped body (10) extending along the direction (X), said ring-shaped body comprising an inner surface and an outer surface, the inner surface of the overmolding member (20) being in contact with the side wall of the rigid body (10) over an outer part (111) of said rigid body (10) extending continuously beyond the inner end (101) of the rigid body (10), the ring-shaped body of the overmolding member (20) extending continuously beyond the inner end (101) of the rigid body (10) and being closed by the sealing portion (21) such that the sealing portion (21) is facing the inner end (101), a space being free between the sealing portion (21) of the overmolding member (20) and the inner end (101) of the rigid body, the adhesion portion being at the level of the outer surface of the overmolding member (20)
- molding said overmolding member (20) by pouring the molding product (33) between the first and second mold (31, 32),
- removing the system of molds (3).

13. Method for manufacturing a port according to claim 12, wherein the body (10) is obtained during a prior molding step.

14. Method for manufacturing a system (2) for dispensing content between the inside of a container and the outside of a container by means of a needle, for which are provided:
- a port (1) and a container (40) as described in any of claims 1 to 11,
the method comprising as steps:
- the overmolding member (20) is partly inserted into the container (40) through the opening,
- the wall of the container at the level of its opening is welded to the adhesion portion of the overmolding member (20) by applying a high-frequency electromagnetic field through the rigid body (10).
